Europäisches Patentamt

European Patent Office

Office européen des brevets

(11) Veröffentlichungsnummer: **0 432 758 A2**

# (12) EUROPÄISCHE PATENTANMELDUNG

(21) Anmeldenummer: 90123961.6

(22) Anmeldetag: **12.12.90**

(51) Int. Cl.5 **C12M 1/40**

(30) Priorität: **13.12.89 DE 3941162**

(43) Veröffentlichungstag der Anmeldung:
**19.06.91 Patentblatt 91/25**

(84) Benannte Vertragsstaaten:
**AT BE CH DE DK ES FR GB GR IT LI LU NL SE**

(71) Anmelder: **Gesellschaft für
biotechnologische Forschung mbH (GBF)
Mascheroder Weg 1
W-3300 Braunschweig-Stöckheim(DE)**

(72) Erfinder: **Dremel, Bernd
Mascheroder Weg 1
W-3300 Braunschweig(DE)**
Erfinder: **Hanisch, Detlef
Mascheroder Weg 1
W-3300 Braunschweig(DE)**

(74) Vertreter: **Boeters, Hans Dietrich, Dr. et al
Boeters & Bauer Bereiteranger 15
W-8000 München 90(DE)**

(54) Sensoranordnung für die Fliessinjektionsanalyse.

(57) Die Erfindung bezieht sich auf eine Sensoranordnung für die Fließinjektionsanalyse, umfassend einen Enzymreaktor und einen chemisch/physikalischen Sensor, bei dem die Sensoranordnung als ein abgeschlossenes Modul ausgeführt ist und ein Durchflußkanal großer Länge in dem Modul untergebracht ist. Mittels dieser Sensoranordnung werden im Enzymreaktor entstandene oder verbrauchte Gase ohne störenden Gasaustausch mit der Umgebung detektiert.

Fig. 1

## SENSORANORDNUNG FÜR DIE FLIESSINJEKTIONSANALYSE

Die Erfindung bezieht sich auf eine Sensoranordnung für die Fließinjektionsanalyse, umfassend einen Enzymreaktor und einen chemisch/physikalischen Sensor. Unter Enzymreaktor sollen hier sämtliche immobilisierten biologischen Komponenten wie Enzyme, Antikörper oder dergleichen verstanden werden, die eine bestimmte Substratspezifität gewährleisten können.

Die Fließinjektionsanalyse (FIA) ist ein naßchemisches Analyseverfahren, das sich in den letzten Jahren zu einer wertvollen und häufig angewandten Methode entwickelt hat. Bei der Fließinjektionsanalyse wird die zu analysierende Probe in eine Strömung einer geeigneten Flüssigkeit (Trägerflüssigkeit) injiziert und zusammen mit dieser einem Detektionssystem zugeführt. Im Detektionssystem wird der Probendurchgang festgestellt und aus dem Ausgangssignal in einer Auswerteeinrichtung der Analytgehalt der Probe quantitativ erfaßt. Zwar ist die Fließinjektionsanalyse kein kontinuierliches Analyseverfahren, jedoch ist die Wiederholrate der Einzelmessungen im allgemeinen so hoch, daß das Verfahren für viele Anwendungsfälle als quasi-kontinuierliches Verfahren angesehen werden kann.

Die Fließinjektionsanalyse ermöglicht somit die Übertragung herkömmlicher analytischer Labortechniken auf einen kontinuierlichen Fließprozeß. Es können vollautomatisch Pipettier-, Verdünnungs- und Mischvorgänge sowie chemische Reaktionen oder Messungen mittels Elektroden oder Optoden durchgeführt werden.

Als Detektoren sind jegliche Einrichtungen geeignet, die die chemische Nachweisreaktion quantitativ in eine elektrisches Signal umwandelt. Detektoren können beispielsweise chemisch/ physikalische Sensoren bzw. Transducer wie Photometer, Fluorimeter, Refraktometer, Lumineszenzdetektoren, Trübungsmesser, pH- und ionensensitive Elektroden, voltametrische und amperometrische Detektoren, Leitfähigkeitsmesser, Thermistoren, Halbleiterstrukturen (FETs) etc. sein.

Voraussetzung für eine fehlerfreie Analyse ist, daß in dem Flüssigkeitsstrom keine Luftblasen vorhanden sind, denn hierdurch kommt es häufig zu völlig falschen Analyseergebnissen. Das Injektionsvolumen wird vermindert, es kommt zu Mischungs- und Reaktionsfehlern und im Detektionssystem zu Meßfehlern. Gegenmaßnahmen zur Verhinderung oder Beseitigung von Luftblasen in Fließinjektionssystemen bestehen darin, daß die verwendeten Flüssigkeiten entgast werden, was relativ aufwendig und nicht ungefährlich ist. Gegen noch im System vorhandene Luftblasen werden gemäß einem neueren Vorschlag Luftblasenfallen verwendet, die im Fließsystem enthaltene Luftblasen aufhalten und forttransportieren.

Die Fließinjektionsanalyse ist auch nicht auf naßchemische Nachweise beschränkt. Durch den Einsatz von Enzymen in gelöster oder immobilisierter Form, von immobilisierten Antigenen/Antikörpern, von Organellen und Mikroorganismen läßt sich der Anwendungsbereich der Fließinjektionsanalyse stark erweitern.

Die Verwendung von Enzymen in der Fließinjektionsanalyse ermöglicht eine spezifische Bestimmung von Analyten auch in komplexen Medien. Gewöhnlich werden die Enzyme immobilisiert, d.h. in einem Enzymreaktor verwendet, der die Erfassung enzymatischer Umsetzungen durch den Analyten, beispielsweise den Verbrauch von Edukten wie z.B. von Sauerstoff und die Bildung von Produkten wie beispielsweise von Kohlendioxid oder von Säuren ermöglicht.

Zur Erzielung genauer Ergebnisse der Analyse wurden bisher die biologische Komponente direkt auf dem chemisch/physikalischen Sensor aufgebracht und der chemisch/physikalische Sensor (Transducer) in einem Bauteil zusammengefaßt. Die Anordnung ist dabei derart, daß die Analyte durch die Enzymschicht zu dem chemisch/physikalischen Sensor diffundieren müssen. Durch die große räumliche Nähe wird verhindert, daß die Konzentration des von dem chemisch/physikalischen Sensor zu erfassenden Analyten durch Dispersion oder Gasaustausch mit der Umgebung schnell abnimmt. Um jedoch das Ansprechverhalten und den linearen Meßbereich nicht unerwünscht zu verändern, kann bei gegebener Geometrie des Bauteils nur eine beschränkte Enzymmenge, d.h. eine Enzymschicht mit bestimmter Dicke, verwendet werden. Hierdurch ist die Sensoranordnung sehr störempfindlich.

Es ist des weiteren eine Sensoranordnung für die Fließinjektionsanalyse entwickelt worden, bei der die biologische Komponente vom chemisch/physikalischen Sensor getrennt ist. Dieser Aufbau ist insbesondere für Biosensoren mit Enzymen geringer Aktivität und/oder Stabilität vorteilhaft. Durch die räumliche Entkopplung kann anders als bei der Schichtenanordnung von biologischer Komponente und Transducer eine größere Enzymmenge verwendet werden. Der so erzielte Enzym überschuß bewirkt eine Verringerung der Empfindlichkeit des Systems in bezug auf Störionen (z.B. Schwermetallionen) und schwankende pH-Werte. Ein Nachteil der Entkopplung der biologischen Komponente vom chemisch/physikalischen Sensor besteht jedoch darin, daß in der Sensoranordnung die zu erfassenden Gase entweichen können, so daß diese von dem nachgeschalteten chemisch/ physikalischen Sensor nur unvollständig detektiert werden. Dies tritt insbesondere bei niedrigen Fließgeschwindigkeiten auf.

Der Erfindung liegt die Aufgabe zugrunde, eine Sensoranordnung für die Fließinjektionsanalyse, umfassend einen Enzymreaktor und einen chemisch/physikalischen Sensor, zu schaffen, bei dem beide in einem Bauteil zusammengefaßt sind und die Verwendung einer ausreichend großen Enzymmenge ermöglicht ist.

Diese Aufgabe ist durch die Erfindung bei einer Sensoranordnung mit den Merkmalen des Anspruchs 1 gelöst. Vorteilhafte Weiterbildungen der erfindungsgemäßen Sensoranordnung sind Gegenstand der Unteransprüche.

Eine erfindungsgemäße Sensoranordnung für die Fließinjektionsanalyse umfaßt somit einen Enzymreaktor und einen chemisch/physikalischen Sensor und ist als ein Modul ausgeführt, wobei in dem Modul ein Durchflußkanal großer Länge untergebracht ist. Bei dieser integrierten Bauweise kommt es zu keiner störenden Diffusion von Gasen wie z.B. von Sauerstoff und Kohlendioxid durch die Wandungen, selbst wenn die Fließgeschwindigkeiten niedrig sind. Es kommt somit zu keinem störenden Gasaustausch zu detektierender Gase und der Umgebung, denn die in dem Biosensor entstandenen oder verbrauchten Gase bleiben in dem Modul, das auch den chemisch/physikalischen Sensor enthält, so daß es nicht zu Detektionsverfälschungen kommen kann. Die Verwendung eines Durchflußkanals mit großer Länge ermöglicht es, mit hohen Fließgeschwindigkeiten zu arbeiten, wodurch der Nachteil der eventuellen Bildung oder des Hängenbleibens von Luftblasen auf ein Minimum herabgesetzt ist. Ein weiterer Vorteil des Modulaufbaus besteht darin, daß die Sensoranordnung leicht ausgetauscht werden kann, ebenso die Enzymreaktoren. Bei der erfindungsgemäßen Sensoranordnung kann vorteilhaft eine größere Enzymmenge verwendet werden, als dies bei der direkten Immobilisierung der biologischen Komponente in einer Schichtanordnung direkt auf dem Transducer der Fall ist, bei der das Erreichen eines Enzymüberschusses nur bedingt erreicht werden kann, da Diffusionsvorgänge die Ansprechzeiten und Meßbereiche des Sensors unerwünscht beeinträchtigen können. Der erfindungsgemäße Enzymreaktor kann innerhalb gewisser Grenzen sehr flexibel dimensioniert werden.

Eine besonders vorteilhafte Ausbildung des Durchflußkanals besteht in dessen Mäanderform, wodurch eine besonders große Kanallänge raumsparend vorgesehen werden kann. Es werden ferner Störungen durch Luftblasen verringert, da diese durch die hohe Fließgeschwindigkeit herausgespült werden.

Der Enzymreaktor ist in der erfindungsgemäßen Sensoranordnung vorzugsweise als austauschbare Einsetzkartusche ausgebildet. Dies ermöglicht es, den Enzymreaktor bei Bedarf einfach auszuwechseln, während die langlebigeren chemisch/physikalischen Sensoren wie beispielsweise Sauerstoff- und Kohlendioxidoptoden im Modul bleiben. Hierdurch sind die Analysekosten deutlich herabgesetzt. Ein weiterer Vorteil dieser Anordnung besteht darin, daß ein rascher Wechsel bzw. Umbau zur Durchführung einer anderen Analysemethode ermöglicht ist.

Ein besonders einfacher Austausch der Einsetzkartusche ergibt sich, wenn die Einsetzkartusche in dem Modul eingeschraubt ist.

Bei einem bevorzugten Ausführungsbeispiel der erfindungsgemäßen Sensoranordnung ist der Gehäuseteil des Enzymreaktors aus Plexiglas (Polytetrafluorethylen) oder aus klarem PVC (Polyvinylchlorid). Die Herstellungskosten sind günstig.

Zweckmäßig weist das Modul eine den Durchflußkanal enthaltende Durchflußkammer auf, an der eine Halterung des chemisch physikalischen Sensors klemmbefestigt ist. Dieser Aufbau kann selbstdichtend ausgeführt werden, so daß keine Gummidichtung mit entsprechenden Nachteilen wie z.B. Versprödung oder Verdrillung erforderlich ist. Die Abdichtung erfolgt durch die Klemmung, d.h. das Anpressen der Halterung des chemisch physikalischen Sensors an den Durchflußkammerteil.

Eine besonders einfach Ausführung der Klemmbefestigung besteht in einer Anordnung mit Überwurfmutter und Gegenschraube.

Vorzugsweise umfaßt der chemisch physikalische Sensor einen mit dem Durchflußkanal in Kontakt stehenden Geber, der optische Signale ausgibt, ein transparentes Fenster und einen hinter dem Fenster angeordneten optischen Aufnehmer. Bei dieser Anordnung ist die Meßwerterfassung von der Meßwertübertragung getrennt, wobei letztere durch die Entkopplung (Fenster) medienunabhängig ist.

Zweckmäßig ist der optische Aufnehmer ein Lichtfaserbündel, das raumsparend angeordnet werden kann.

Das optische Fenster ist vorzugsweise aus Saphir oder Quarz.

Eine vorteilhafte Ausbildung des optischen Gebers ist eine Optode, die aus einer Trägerfolie und einer auf diese auf gebrachten Indikatorschicht besteht. Diese Anordnung ist hochempfindlich und zugleich raumsparend.

Als Trägerfolie wird vorzugsweise eine Polyesterfolie auf Basis von Ethylenglykol und Terephthalsäure, d.h. eine Mylarfolie, verwendet, wobei die Indiaktorschicht vorzugsweise eine Silikonschicht mit Fluoreszenzindikator ist.

Ein besonders einfacher Aufbau ergibt sich dadurch, daß der Geber und das optische Fenster mit der Halterung fluiddicht angedrückt sind, wobei der Geber eine Dichtschicht aufweist und eine Verschiebesicherung für den Geber vorgesehen ist. Hierdurch werden keine zusätzlichen Teile benötigt und die Montage ist vereinfacht.

Die Erfindung wird im folgenden anhand eines bevorzugten Ausführungsbeispiels und der Zeichnung weiter beschrieben. In der Zeichnung zeigen:

Fig. 1    eine zum Teil fortgebrochene Ansicht der Sensoranordnung mit zusammenschraubtem Biosensor und chemisch/physikalischem Sensor,

Fig. 2    eine Seiten- und Vorderansicht der den Durchflußkanal enthaltenden Durchflußkammer der Sensoranordnung von Fig. 1, wobei der Enzymreaktor und Halterungsteile fortgelassen sind,

Fig. 3    eine Vorder- · und Seitenansicht der Überwurfmutter und Gegenschraube,

Fig. 4    eine Seiten- und Vorderansicht der Halterung für den chemisch/physikalischen Sensor,

Fig. 5    eine Vorder- und Seitenansicht der Fensterhalterung,

Fig. 6    Teilansichten des Fensters, der bei dem chemisch/ physikalischem Sensor verwendeten Optode, einer Justierschraube zur Halterung eines als optischer Aufnehmer verwendeten Lichtfaserbündels, eines Paßstiftes zur Verschiebesicherung des mit dem Durchflußkanal in Kontakt stehenden Gebers und des Enzymreaktors und

Fig. 7    eine Seitenansicht der Optode.

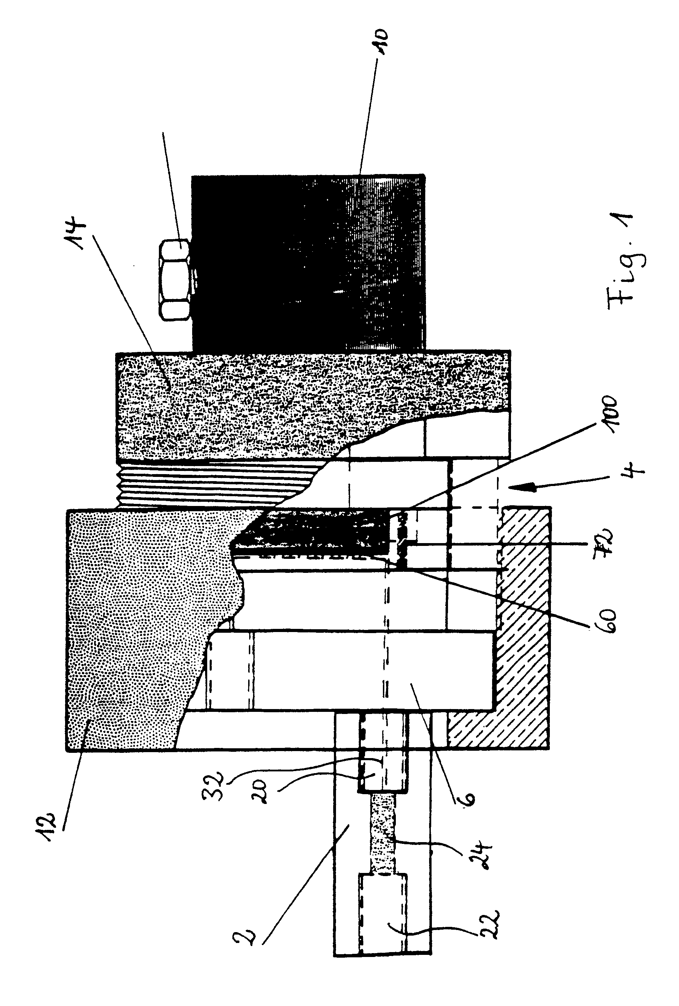

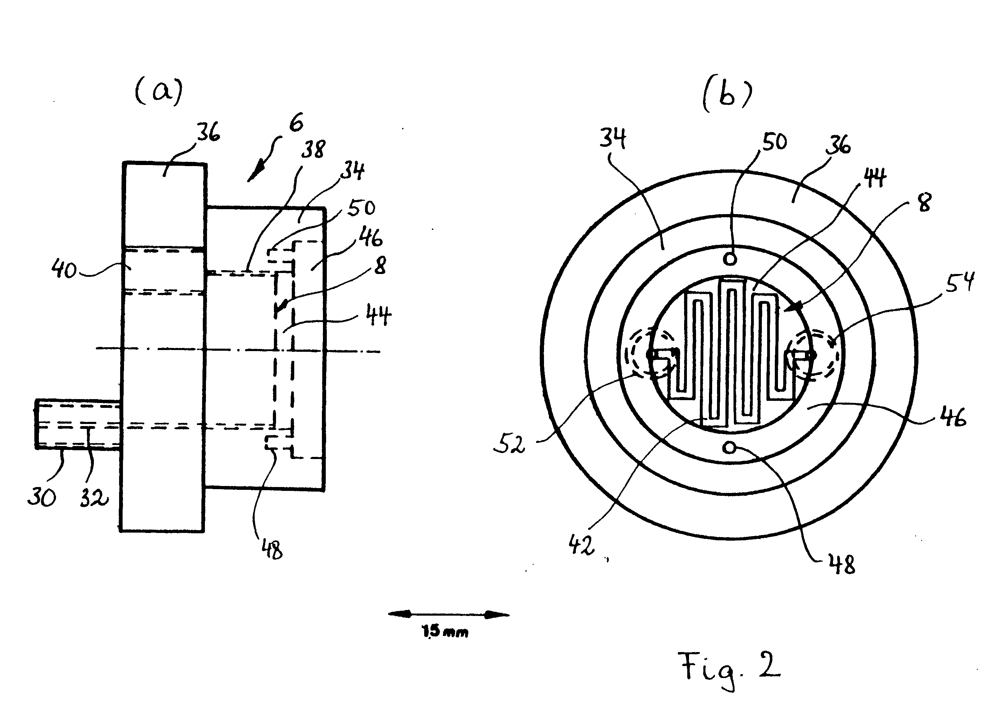

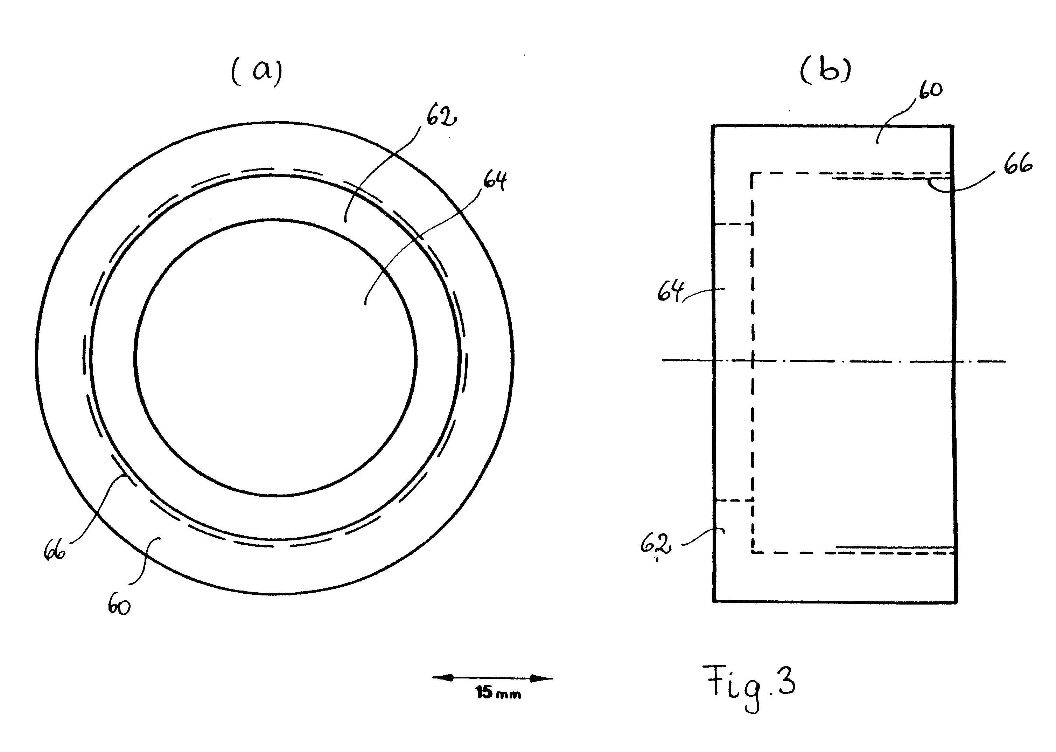

In Fig. 1 ist ein Ausführungsbeispiel der erfindungsgemäßen Sensoranordnung für die Fließinjektionsanalyse dargestellt. Die allgemein ein Gehäuse aus schwarzem PVC (Polyvinylchlorid) aufweisende Sensoranordnung umfaßt einen Enzymreaktor 2 und einen chemisch/physikalischen Sensor 4, die beide mit einem in einer Durchflußkammer 6 enthaltenen Durchflußkanal 8 in Kontakt stehen. Der chemisch/physikalische Sensor 4 weist eine Halterung 10 auf, die mittels einer Überwurfmutter 12 und einer Gegenschraube 14 aus VA-Stahl an der Durchflußkammer 6 klemmbefestigt ist.

Der Enzymreaktor 2 umfaßt ein Gehäuse aus Plexiglas (Acrylglas) mit zentralen Bohrungen 20, 22 und einem diese verbindenden zentralen Aufnahmezylinder 24. Mittels der Bohrung 20 ist der Enzymreaktor 2 direkt auf einen mit Außengewinde versehenen Stutzen 30 der Durchflußkammer 6 aufgeschraubt, der eine zentrale Bohrung 32 als Endabschnitt des Durchflußkanals 8 aufweist. In dem Aufnahmezylinder 24 des Enzymreaktors 2 ist der eigentliche Aufbau der biologischen Komponente untergebracht. Die Bohrung 22 ist zum Anschluß und zur Durchführung von Meßleitungen, beispielsweise einer lichtleitenden Einzelfaser oder dergl. vorgesehen.

In Fig. 2 ist die Durchflußkammer 6 mehr im einzelenen dargestellt. Sie umfaßt eine zylindrische Kammer 34 und einen Flansch 38, der auf der Außenseite den Stutzen 30 trägt, wobei sich die Bohrung 32 des Stutzens 30 durch den Flansch 36 und die Kammer 34 bis zu einer noch zu beschreibenden Optode 70 fortsetzt. Gegenüber der Bohrung 32 weist die Kammer 34 eine weitere Bohrung 38 auf, die in eine größere Bohrung 40 im Flansch 36 übergeht. Die in Höhe der Optode 70 endenden Bohrungen 32, 38 sind durch einen Mäanderkanal 42 miteinander verbunden. Benachbart dem Mäanderkanal weist die Kammer 34 eine zylindrische Ausnehmung 44 mit einem Durchmesser auf, der etwa dem Abstand der Bohrungen 32 und 38 voneinander entspricht. Die Ausnehmung 44 ist zur Aufnahme der Optode 70 vorgesehen. Die Ausnehmung 44 erweitert sich in eine weitere zylindrische Ausnehmung 46 mit größerem Durchmesser, die auf ihrem Boden mit zwei axial gerichteten Bohrungen 48, 50 zur Aufnahme von noch zu erläuternden Paßstiften 72 vorgesehen sind. Ein- und Auslaßbohrungen 52, 54 als Anschluß für den Durchflußkanal 8 sind in Fig. 2 lediglich schematisch dargestellt.

In Fig. 3 ist die Überwurfmutter 12 in Vorder- und Seitenansicht dargestellt. Die Überwurfmutter umfaßt einen zylindrischen Ringkörper 60 mit einem ebenen Boden 62, der eine zentrale Öffnung 64 aufweist. Auf der Innenseite weist der Ringkörper 60 ein Gewinde 66 auf, mittels dessen die Überwurfmutter 12 und die Gegenschraube 14 miteinander verschraubt werden.

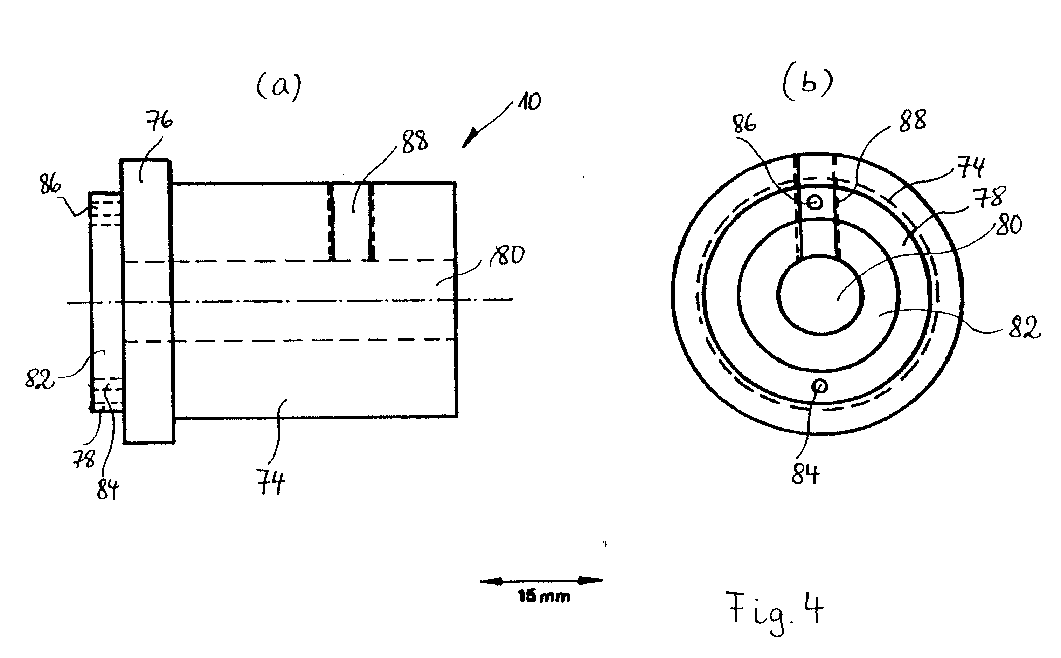

Fig. 4 zeigt eine Seiten- und Vorderansicht der Halterung 10 des chemisch/physikalischen Sensors 4. Die Halterung 10 besteht aus einem Zylinderkörper 74, der im Bereich des vorderen Endes zu einem Zylinderring 76 mit größerem Durchmesser erweitert ist, an den sich zur Vorderseite ein zylindrischer Ansatz 78 mit einem Durchmesser anschließt, der etwa dem Durchmesser der Ausnehmung 46 entspricht. Der Zylinderkörper 74 weist eine zentrale Bohrung 80 auf. Die Bohrung 80 geht in eine Ausnehmung 82 des Ansatzes 78 über, wobei der Durchmesser des Ansatzes 78 dem Durchmesser der Ausnehmung 44 entspricht. Im Ansatz 78 sind ferner Bohrungen 84, 86, entsprechend den Bohrungen 48, 50 zur Aufnahme der Paßstifte 72 vorgesehen. Etwa im mittleren Bereich weist der Zylinderkörper 74 eine sich nach außen erstreckende, gewindeversehene radiale Bohrung 88 auf, die zur Aufnahme einer Justierschraube aus VA-Stahl 90 vorgesehen ist. Die Justierschraube 90 ist zur Fixierung eines in die Halterung 10 einge-führten, nicht dargestellten Lichtfaserbündels vor-gesehen, das als Signalleitung des chemisch/physikalischen Sensors 4 vorgesehen ist.

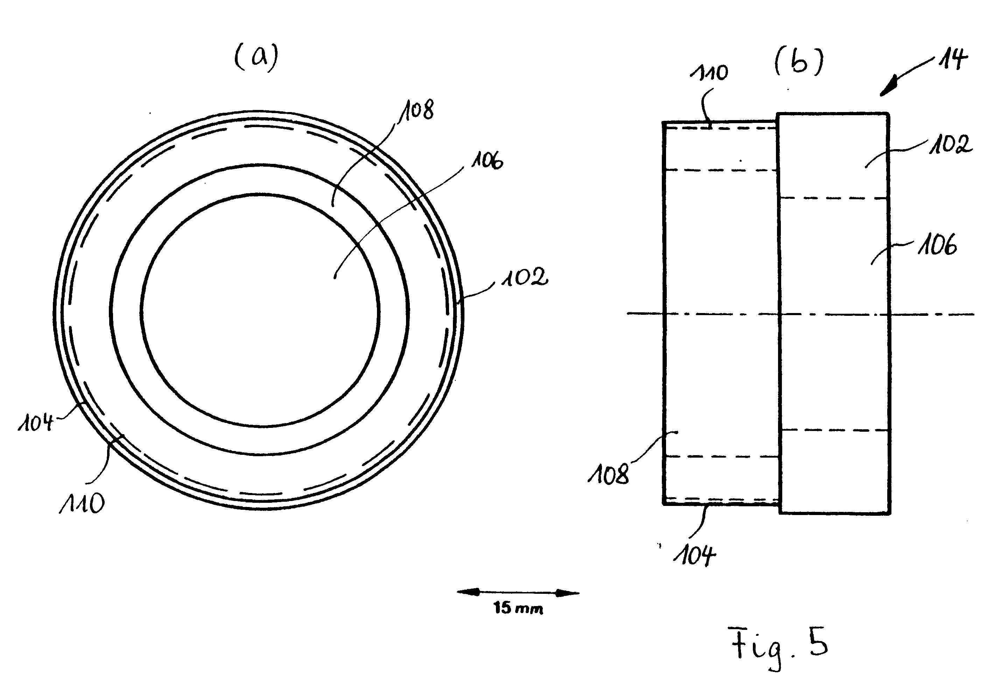

In Fig. 5 ist eine Vorder- und Seitenansicht der Gegenschraube 14 dargestellt, die gleichzeitig die Funktion einer Halterung für ein noch zu beschreibendes Fenster 100 darstellt. Die Gegenschraube 14 besteht aus einem Zylinderring 102, der in einen Zylinderring 104 mit etwas kleinerem Durchmesser übergeht. Der erstere Zylinderring 102 weist eine zentrale Bohrung 106 auf, deren Durchmesser dem Außendurchmesser des Zylinderkörpers 64 entspricht, und der andere Zylinderring 104 weist eine zentrale Bohrung 108 auf, deren Innendurchmesser dem Außendurchmesser des Zylinderrings 76 entspricht. Der Zylinderring 104 weist des weiteren ein Außengewinde 110 auf, das dem Gewinde 66 des Ringkörpers 60 entspricht. Durch Zusammenschrauben der Übberwurfmutter 12 und ihrer Gegenschraube 14 werden die dazwischen angeordnete Halterung 10 und die Durchflußkammer 6 zusammengedrückt.

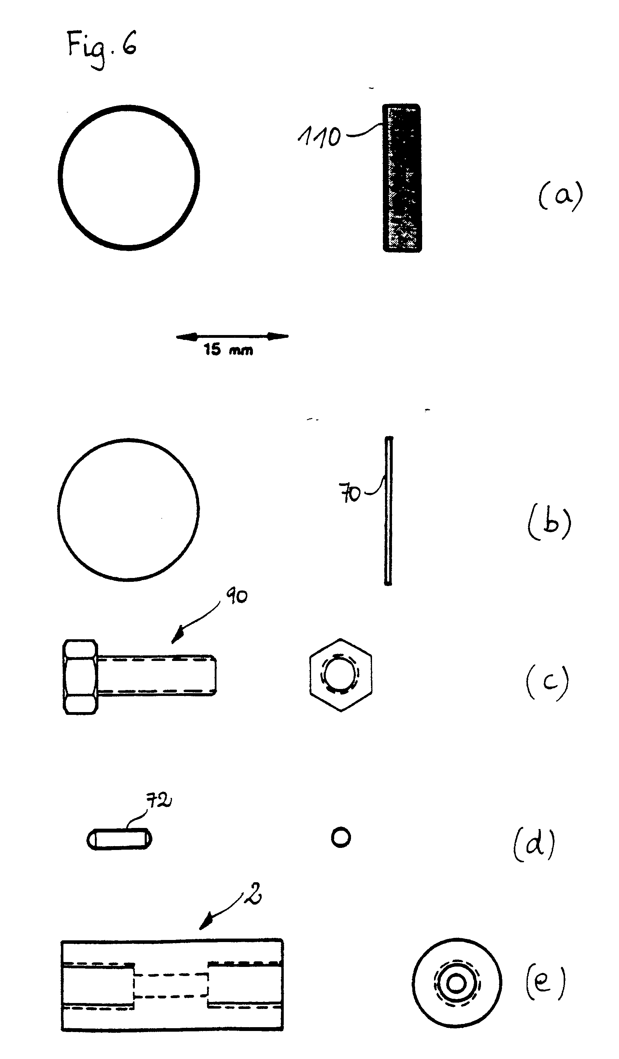

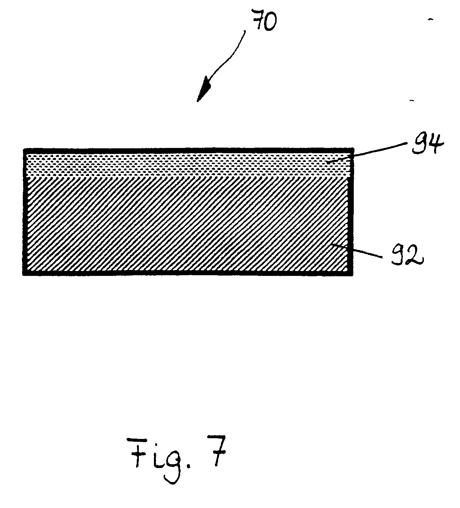

Fig. 6 zeigt Einzeldarstellungen von Teilen der Sensoranordnung. In Fig. 6 (a) ist das optische Fenster 100 veranschaulicht, das im dargestellten Ausführungsbeispiel aus Saphir ist. Die auf dem optischen Fenster 100 liegende Optode 70 ist in Fig. 6 (b) veranschaulicht und in Fig. 7 in größerem Maßstab dargestellt. Die Optode 70 besteht aus einer Trägerfolie 92, die im gezeigten Ausführungsbeispiel eine Mylarfolie ist, d.h. eine Polyesterfolie auf Basis von Ethylenglykol und Terephtalsäure. Die Trägerfolie 92 ist mit einer Indikatorschicht 94 aus Silikon und einem darin enthaltenen Fluoreszenzindikator versehen. Die von der Indikatorschicht 94 ausgesandten optischen Signale werden durch das optische Fenster 100 auf das Lichtfaserbündel gegeben.

In Fig. 6 (c) ist die Justierschraube 90 zum Fixieren des Lichtfaserbündels in der Halterung 10 veranschaulicht. Ein zylindrischer Paßstift 72, der als Verschiebesicherung für die Optode 70 vorgesehen ist, ist in Fig. 6 (d) veranschaulicht. Der Paßstift 72 ist in den Bohrungen 48, 50 aufgenommen.

In Fig. 6 (e) ist schließlich der Biosensor 2 dargestellt.

## Ansprüche

1. Sensoranordnung für die Fließinjektionsanalyse, umfassend einen Enzymreaktor und einen chemisch/physikalischen Sensor, dadurch gekennzeichnet, daß die Sensoranordnung als ein abgeschlossenes Modul ausgeführt ist und ein Durchflußkanal (8) großer Länge in dem Modul untergebracht ist.

2. Sensoranordnung nach Anspruch 1, dadurch gekennzeichnet, daß der Durchflußkanal (8, 42) mäanderförmig ist.

3. Sensoranordnung nach Anspruch 1 oder 2, dadurch gekennzeichnet, daß der Enzymreaktor (2) als austauschbare Einsetzkartusche ausgebildet ist.

4. Sensoranordnung nach Anspruch 3, dadurch gekennzeichnet, daß die Einsetzkatusche (2) in dem Modul eingeschraubt ist.

5. Sensoranordnung nach einem der Ansprüche 1 bis 4, dadurch gekennzeichnet, daß der Gehäuseteil des Biosensors (2) aus Plexiglas (Polytetrafluorethylen) oder aus klarem PVC (Polyvinylchlorid) ist.

6. Sensoranordnung nach einem der Ansprüche 1 bis 5, dadurch gekennzeichnet, daß das Modul eine den Durchflußkanal (8) enthaltende Durchflußkammer (6) aufweist, an der eine Halterung (10) des chemisch.physikalischen Sensors (4) klemmbefestigt ist.

7. Sensoranordnung nach Anspruch 6, dadurch gekennzeichnet, daß als Klemmbefestigung eine Überwurfmutter (12) und eine Gegenschraube (14) vorgesehen sind.

8. Sensoranordnung nach einem der Ansprüche 1 bis 7, dadurch gekennzeichnet, daß der chemisch.physikalische Sensor (4) einen mit dem Durchflußkanal (8) in Kontakt stehenden Geber (70), der optische Signale ausgibt. ein transparentes Fenster (100) und einen hinter dem Fenster angeordneten optischen Aufnehmer umfaßt.

9. Sensoranordnung nach Anspruch 8. dadurch gekennzeichnet, daß der optische Aufnehmer ein Lichtfaserbündel ist.

10. Sensoranordnung nach Anspruch 8 oder 9. dadurch gekennzeichnet, daß das optische Fenster aus Saphir oder Quarz ist.

11. Sensoranordnung nach einem der Ansprüche 8 bis 10, dadurch gekennzeichnet, daß der optische Geber (70) eine Optode ist, die aus einer Trägerfolie (92) und einer auf diese aufgebrachten Indikatorschicht (94) besteht.

12. Sensoranordnung nach Anspruch 11, dadurch gekennzeichnet, daß die Trägerfolie (92) eine Polyesterfolie auf Basis von Äthylenglykol und Terephthalsäure (Mylarfolie) ist und oder die Indikatorschicht (94) eine Silikonschicht mit Fluoreszenzindikator ist.

13. Sensoranordnung nach einem der Ansprüche 8 bis 12, dadurch **gekennzeichnet,** daß der Geber (70) und das optische Fenster (100) mit der Halterung (10) fluiddicht angedrückt sind, wobei der Geber eine Dichtschicht aufweist und eine Verschiebesicherung (48, 50, 52) für den Geber vorgesehen ist.

Fig. 1

(a)

(b)

15 mm

Fig. 2

EP 0 432 758 A2

( a )

( b )

15 mm

Fig. 3

EP 0 432 758 A2

(a)

(b)

15 mm

Fig. 4

EP 0 432 758 A2

(a) (b)

108

106

110

102

104

110

104

106

102

14

108

15 mm

Fig. 5

EP 0 432 758 A2

# Fig. 6

110

(a)

← 15 mm →

70

(b)

90

(c)

72

O

(d)

2

(e)

Fig. 7